# EUROPÄISCHE PATENTSCHRIFT

(11) **EP 0 498 240 B1**
(45) Veröffentlichungstag und Bekanntmachung des Hinweises auf die Patenterteilung: **07.12.1994**
(21) Anmeldenummer: 92101200.1
(22) Anmeldetag: 25.01.1992
(51) Int. Cl.: C07C 309/42, G01N 33/78

(54) **Diphenylmethanderivate, Verfahren zu deren Herstellung und ihre Verwendung zur Verdrängung von Jodthyroninen und diese bindenden Proteinen**
Derivatives of diphenylmethane, process for their preparation and their use in supplanting iodothyronines and the proteins binding them
Dérivés de diphénylméthane, procédé pour leur préparation et leur utilisation pour le refoulement de iodothyronines et des protéines les liants

(30) Priorität: 02.02.1991 DE 4103167
(43) Veröffentlichungstag der Anmeldung: 12.08.1992
(73) Patentinhaber: BOEHRINGER MANNHEIM GMBH, 68298 Mannheim (DE)
(72) Erfinder: Freitag, Helmut, Dr. rer. nat., W-6940 Weinheim (DE); Knappe, Wolfgang-Reinhold, Dr. rer.nat.,Dipl.Chem., W-6700 Ludwigshafen (DE); Eikmeier, Heino, Dr. rer.nat., Dipl.-Biol., W-6143 Lorsch (DE); Weckerle, Wolfgang F., Dr. rer.nat.,, W-6718 Grünstadt (DE)

(56) Entgegenhaltungen:
- EP-A- 0 133 464
- WO-A-91/06589

## Beschreibung

Die Erfindung betrifft Diphenylmethanderivate der allgemeinen Formel I 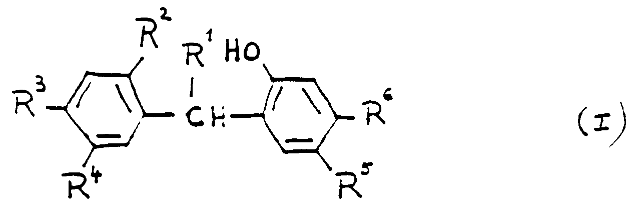
wobei
- R¹ und R²,: die gleich oder verschieden sind, Wasserstoff oder Hydroxy,
- R³: Wasserstoff oder Alkoxy,
- R⁴: Wasserstoff, CO₂H, SO₃H, PO₃H₂ oder ein Salz der entsprechenden Säure,
- R⁵: CO₂H, SO₃H, PO₃H₂ oder ein Salz der entsprechenden Säure und
- R⁶: Alkoxy
bedeuten sowie ein Verfahren zur Herstellung solcher Verbindungen.

Die Erfindung betrifft insbesondere die Verwendung solcher Verbindungen zur Verdrängung von Jodthyroninen von diesen bindenden Proteinen, speziell in einem Verfahren zur Bestimmung eines Jodthyronins in Gegenwart jodthyroninbindenden Proteins in einer flüssigen Probe durch Zugabe einer Substanz, die an Protein gebundenes Jodthyronin von dort verdrängt und freisetzt und Bestimmung des gesamten Jodthyronins in der Probe, insbesondere mittels einer immunologischen Methode. Außerdem betrifft die Erfindung die Verwendung der erfindungsgemäßen Verbindungen in einem entsprechenden Reagenz zur Bestimmung von Jodthyronin.

Jodthyronine im Sinne der vorliegenden Erfindung sind insbesondere solche, die für die klinische Diagnostik von Interesse sind. Solche Jodthyronine sind vor allem 3,5,3',5'-Tetrajodothyronin (Thyroxin; T4), 3,5,3'-Trijodothyronin (T3), 3,3',5'-Trijodothyronin und 3,3'-Dijodthyronin. Zur Untersuchung der Schilddrüsenfunktion spielt vor allem die quantitative Bestimmung der Konzentration von T4 und/oder T3 in Blut oder von Blut abgeleiteten Proben, wie Plasma oder Serum eine sehr wichtige Rolle. In Blut, Plasma oder Serum ist ein Großteil des dort vorkommenden Jodthyronins an Protein gebunden. Solche jodthyroninbindenden Proteine sind beispielsweise Albumin, thyroxinbindendes Präalbumin und vor allem "tyroxine binding globulin" (TBG).

Um den Gesamtgehalt einer Blutprobe an Jodthyronin bestimmen zu können, muß bei einer Reihe von Methoden proteingebundenes Jodthyronin von dem entsprechenden Trägerprotein abdissoziiert werden, um dann das frei vorliegende Jodthyronin bestimmen zu können. Zur Freisetzung von proteingebundenem Jodthyronin ist es üblich, sogenannte "Verdrängungsreagenzien" zuzusetzen, die Jodthyronin von der entsprechenden Bindungsstelle am Protein verdrängen und selbst an das Protein binden. Solche Verdrängungsreagenzien sind beispielsweise aus den europäischen Patentanmeldungen Nr. 0 078 477 und 0 133 464 bekannt

EP-A-0 078 477 beschreibt als verdrängend wirkende Substanzen solche der allgemeinen Formel
Z₁-Y-Z₂, wobei Z₁ und Z₂ Phenylgruppen darstellen, die durch Halogen, Alkyl und/oder Alkoxy substituiert sein können und von denen ein Phenylring einen Carbonsäure oder Sulfonsäurerest trägt. Y kann Sauerstoff, Imin, Schwefel, Methylen oder Carbonyl sein. Bevorzugt sind jedoch Phenylessigsäurederivate, die durch Chlor substituiert sind.

In EP-A-0 133 464 wird 2-Hydroxy-4-methoxybenzophenon-5-sulfonsäure sowie Salze dieser Säure als besonders vorteilhaft zur Verdrängung von Jodthyronin von dieses bindendem Protein beschrieben.

An die Freisetzung von zuvor an Protein gebundenem Jodthyronin schließt sich die Bestimmung freien Jodthyronins in der Probe an. In der Regel werden hierzu immunologische Verfahren durchgeführt. Bei solchen immunologischen Verfahren wird Jodthyronin an entsprechende Antikörper gebunden. Um das Ausmaß der Bindung von Jodthyronin an Antikörper, d. h. wieviel Jodthyronin an Antikörper gebunden wurde, festzustellen, enthalten immunologische Bestimmungsreagenzien Markierungen, die eine quantitative Bestimmung des Jodthyroningehaltes in der zu untersuchenden Probe erlauben. Mögliche Markierungen sind Enzyme, die bestimmte Reaktionen katalysieren und die durch das Ausmaß einer solchen Enzymreaktion anzeigen, wieviel Jodthyronin sich in der zu bestimmenden Probe befindet.

Um den Gesamtgehalt an Jodthyronin in der Probe zuverlässig bestimmen zu können, ist es erforderlich, daß das eingesetzte Verdrängungsreagenz weder die immunchemische Reaktion, d. h. die Reaktion des spezifischen Antikörpers mit Jodthyronin, noch die Aktivität des Markierungsenzyms beeinflußt. Die bisher bekannten Substanzen zur Verdrängung von Jodthyronin von entsprechenden Trägerproteinen zeigen jedoch entweder keine gute Verdrängungswirkung oder sie stören die immunologische Bestimmung von Jodthyronin, indem sie die Jodthyronin-Antikörperreaktion oder die Aktivität eines Markierungsenzyms bei den Konzentrationen, bei denen sie verdrängend wirken, negativ beeinflussen.

Aufgabe der vorliegenden Erfindung war es deshalb, Verbindungen zur Verfügung zu stellen, die Jodthyronin möglichst gut von entsprechenden Bindeproteinen verdrängen und die außerdem immunologische Nachweisreaktionen von Jodthyronin, insbesondere Enzymimmunoassays möglichst wenig störend beeinflussen.

Diese Aufgabe wird durch den Gegenstand der vorliegenden Erfindung, wie er in den Patentansprüchen gekennzeichnet ist, gelöst.

Gegenstand der Erfindung sind Verbindungen der allgemeinen Formel I 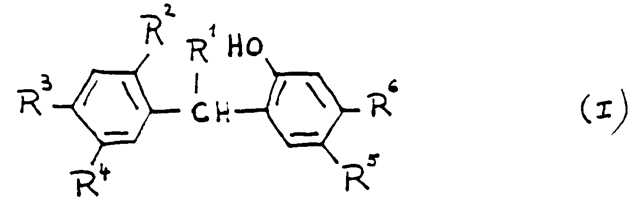
in der
- R¹ und R²,: die gleich oder verschieden sind, Wasserstoff oder Hydroxy,
- R³: Wasserstoff oder Alkoxy,
- R⁴: Wasserstoff, CO₂H, SO₃H, PO₃H₂ oder ein Salz der entsprechenden Säure,
- R⁵: CO₂H, SO₃H, PO₃H₂ oder ein Salz der entsprechenden Säure und
- R⁶: Alkoxy
bedeuten.

Alkoxyreste in der Definition der Verbindungen der allgemeinen Formel I sind insbesondere solche mit 1 bis 6 Kohlenstoffatomen. Besonders bevorzugt sind Alkoxyreste mit 1 bis 3 Kohlenstoffatomen. Ganz besonders bevorzugt ist der Methoxyrest.

Unter einem Salz eines der für die Reste R⁴ und R⁵ als mögliche Bedeutung angegebenen Säurereste werden insbesondere Alkali-, Erdalkali-und Ammoniumsalze verstanden. Unter Alkalisalzen werden Lithium-, Natrium-, Kalium-, Rubidium- und Cäsiumsalze verstanden, wobei Lithium-, Natrium- und Kaliumsalze, vor allem Natrium- und Kaliumsalze, ganz besonders aber Natriumsalze bevorzugt sind. Erdalkalisalze sind solche des Berylliums, Magnesiums, Calciums, Strontiums oder Bariums. Bevorzugt sind Magnesium- und Calciumsalze, wobei Calciumsalze besonders bevorzugt sind. Als Ammoniumsalze können solche des unsubstituierten Ammoniumions, NH₄+, Verwendung finden. Es ist aber auch möglich, solche Ammoniumsalze einzusetzen, bei denen das Ammoniumion durch 1 bis 4 Alkyl-, Aryl- oder Aralkylreste substituiert ist. Hierbei bedeutet "Alkyl" einen geradkettigen oder verzweigten Alkylrest mit 1 bis 6, vorzugsweise 1 bis 4 Kohlenstoffatomen. Beispiele sind die Methyl-, Ethyl-, Propyl-, Isobutyl- oder tert.-Butylgruppe. Bevorzugte Alkylreste als Substituenten des Ammoniumions sind Methyl, Etyhl und n-Propyl. "Aryl" bezeichnet einen Kohlenstoffaromaten- oder Heteroaromatenrest, vorzugsweise einen solchen mit 6 bis 10 Ringatomen, insbesondere die Phenyl- und die Naphthylgruppe, die zusätzlich noch durch Alkyl, Alkoxy oder/und Halogen substituiert sein können. Halogen kann hierbei für die Reste Fluor, Chlor, Brom und Jod stehen. Fluor und Chlor sind bevorzugt. "Alkyl" und "Alkoxy" haben die zuvor gegebenen Bedeutungen. Ein besonders bevorzugter Arylrest in substituierten Ammoniumionen ist der Phenylrest. Ein "Aralkyl"-Rest bedeutet einen Rest, bei dem eine wie vorstehend definierte Alkylgruppe durch einen wie zuvor charakterisierten Arylrest substituiert ist. Bevorzugt ist die Benzylgruppe. Als Salze der erfindungsgemäßen Verbindungen der allgemeinen Formel I sind Alkalisalze in der vorstehend angegebenen Bedeutung ganz besonders bevorzugt.

Bevorzugte Verbindungen der allgemeinen Formel I sind solche, bei denen mindestens einer der möglichen Säurereste R⁴ oder R⁵ eine Sulfonsäuregruppe oder ein Salz einer solchen Säuregruppe ist.

Die erfindungsgemäße Aufgabe wird von Verbindungen der allgemeinen Formel I,
in der
R¹ Wasserstoff oder Hydroxy,
R², R³ und R⁴ Wasserstoff
R⁵ SO₃H oder ein entsprechendes Salz und
R⁶ Alkoxy
bedeuten sowie von solchen Verbindungen der allgemeinen Formel I,
in der
- R¹: Wasserstoff oder Hydroxy,
- R²: Hydroxy,
- R³: Alkoxy,
- R⁴: SO₃H oder ein entsprechendes Salz,
- R⁵: SO₃H oder ein entsprechendes Salz und
- R⁶: Alkoxy
bedeuten, besonders gut gelöst.

Von den als bevorzugt genannten Verbindungen sind wiederum diejenigen am vorteilhaftesten einsetzbar, bei denen R¹ Wasserstoff bedeutet. Von solchen Diphenylmethanderivaten sind insbesondere 2-Hydroxy-4-methoxydiphenylmethan-5-sulfonsäure und entsprechende Salze sowie 2,2'-dihydroxy-4,4'-dimethoxydiphenylmethan-5,5'-disulfonsäure sowie ihre entsprechenden Salze als besonders geeignete Vertreter zu nennen. Erfindungsgemäß hervorragend geeignet ist insbesondere die letztgenannte Säure und ihre Salze, insbesondere das Dinatriumsalz.

Die erfindungsgemäßen Verbindungen sind neu. Sie zeichnen sich insbesondere dadurch aus, daß sie in Wasser und wässrigen Medien, wie Pufferlösungen, gut löslich sind und Jodthyronine, insbesondere Thyroxin, sehr gut von diese bindenden Proteinen, vor allem TBG, verdrängen können. Vor allem zeichnen sie sich dadurch aus, daß sie bei sehr guter Verdrängungswirkung ein deutlich verringertes Störungspotential bei immunologischen Nachweisreaktionen aufweisen, als es bisher aus dem Stand der Technik bekannt war. Hierbei sind insbesondere immunologische Reaktionen auf der Ebene der Jodthyronin-Antikörper-Bindereaktion, als auch enzymatische Reaktionen von Markierungsenzymen mit entsprechenden Substraten zu nennen.

Gegenstand der Erfindung ist auch ein Verfahren zur Herstellung der erfindungsgemäßen Verbindungen. Vorzugsweise werden Verbindungen der allgemeinen Formel I dadurch hergestellt, daß Verbindungen der allgemeinen Formel II 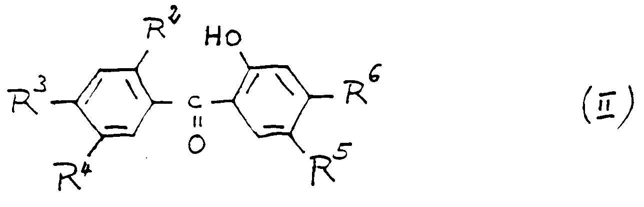
in der
- R²: Wasserstoff oder Hydroxy,
- R³: Wasserstoff oder Alkoxy,
- R⁴: Wasserstoff, CO₂H, SO₃H, PO₃H₂ oder ein Salz der entsprechenden Säure,
- R⁵: CO₂H, SO₃H, PO₃H₂ oder ein Salz der entsprechenden Säure und
- R⁶: Alkoxy
bedeuten,
reduziert werden. Die Reste haben hierbei die gleichen Bedeutungen, wie sie für Verbindungen der allgemeinen Formel I angegeben sind.

Je nach Wahl der Reduktionsbedingungen ist es möglich, die Bedeutung R¹ in Verbindungen der allgemeinen Formel I zu steuern. So wird bei Reaktion eines Ketons der allgemeinen Formel II mit Natriumcyanoborhydrid unter sauren Bedingungen eine Verbindung der allgemeinen Formel I erhalten, in der R¹ Wasserstoff bedeutet. Bevorzugt wird eine solche Reaktion in wässrigem Medium bei pH 2 bis 4 durchgeführt.

Das gleiche Resultat wird auch erhalten, wenn das Keton der allgemeinen Formel II in Gegenwart von Palladium auf Kohle mit Wasserstoff hydriert wird. Vorzugsweise geschieht dies unter Rühren während mehrerer Stunden in einer Wasserstoffatmosphäre.

Wird ein Keton der allgemeinen Formel II jedoch in Gegenwart von Platin(IV)oxid mit Wasserstoff hydriert, wird hierbei eine Verbindung der allgemeinen Formel I erhalten, in der R¹ Hydroxy bedeutet. Vorteilhafterweise wird auch hier mehrere Stunden lang in einer Wasserstoffatmosphäre gerührt.

Das erfindungsgemäße Verfahren wird vorzugsweise bei Temperaturen zwischen 10 und 40 °C, besonders bevorzugt bei Raumtemperatur, durchgeführt.

Ein weiterer Gegenstand der Erfindung ist die Verwendung von Verbindungen der allgemeinen Formel I zur Verdrängung von Jodthyronin von dieses bindenden Proteinen, insbesondere in einem Verfahren und Reagenz zur Bestimmung von Jodthyronin in Gegenwart jodthyroninbindenden Proteins.

Das erfindungsgemäße Verfahren zur Bestimmung eines Jodthyronins in Gegenwart jodthyroninbindenden Proteins in einer flüssigen Probe durch
Zugabe einer Substanz, die an Protein gebundenes Jodthyronin von dort verdrängt und freisetzt und
Bestimmung des gesamten Jodthyronins in der Probe ist dadurch gekennzeichnet, daß als verdrängende Substanz eine Verbindung der allgemeinen Formel I zu der Probe zugesetzt wird. Die zur Verdrängung von Jodthyronin von dieses bindendem Protein notwendige Konzentration ist selbstverständlich abhängig von der Menge an bindendem Protein in der Probe. In der Praxis hat es sich für menschliche Proben, insbesondere Blut, Plasma oder Serum gezeigt, daß bei Einstellung einer Konzentration der erfindungsgemäßen Substanz der allgemeinen Formel I in der zu untersuchenden Probe von mindestens 6 mmol/l Jodthyronin, vor allem T4 praktisch vollständig von jodthyroninbindendem Protein in der Probe, insbesondere von "thyroxine binding globulin" (TBG) verdrängt wird. Als besonders bevorzugt hat sich eine Konzentration von 8 bis 30 mmol/l erwiesen. Die unteren Grenzen dieses Konzentrationsbereiches sind diejenigen, die zur Erreichung einer praktisch vollständigen Verdrängung von Jodthyronin von seinem entsprechenden bindenden Protein in der Probe nötig sind. Die oberen Grenzen des Konzentrationsbereiches sind vor allem durch wirtschaftliche Überlegungen gesetzt. Mehr Verdrängungsreagenz einzusetzen als notwendig ist, führt zu keiner weiteren verdrängenden Wirkung. In der Praxis hat es sich gezeigt, daß der als bevorzugt angegebene Konzentrationsbereich zur Bestimmung der in-vivo vorkommenden Jodthyroninkonzentrationen optimal ist. Im übrigen liegt es jedoch im Ermessen des Fachmannes, im Einzelfall die optimale Konzentration an Substanz der allgemeinen Formel I zu ermitteln.

Zur Bestimmung des gesamten Jodthyroningehaltes in einer Probe schließt sich an die vorstehend beschriebene Verdrängungsreaktion der Nachweis von Jodthyronin in der Probe an. Dieser kann nach bisher bekannten Methoden durchgeführt werden. Üblicherweise werden immunologische Bestimmungsverfahren angewandt. Unter immunologischen Bestimmungsverfahren oder Immunoassays werden hierbei solche Verfahren verstanden, die auf einer Antigen- oder Hapten-Antikörper-Wechselwirkung beruhen. Als Antikörper können ganze Antikörper (polyklonale oder monoklonale Antikörper) oder entsprechend wirkende Fragmente hiervon, beispielsweise Fab, eingesetzt werden.

Zur Durchführung von Immunoassays muß entweder der eingesetzte Antikörper oder eine der zu bestimmenden Substanz analoge Verbindung detektierbar markiert sein. Erfindungsgemäß eignen sich Verbindungen der allgemeinen Formel I insbesondere zum Einsatz bei solchen Verfahren, in denen Enzyme als Markierungen eingesetzt werden. Solche Bestimmungsverfahren sind als Enzymimmunoassays bekannt (siehe beispielsweise M. Oellerich, J. Clin. Chem. Clin. Biochem. 22, 895 - 904 (1984)). Da die erfindungsgemäßen Verbindungen in den zur Verdrängung von Jodthyronin von dieses bindenden Proteinen notwendigen Konzentrationen weder die Bindung von Jodthyronin an entsprechende Antikörper, noch die Aktivität von für Enzymimmunoassays eingesetzten Enzymen wesentlich beeinträchtigen, wird durch die Bereitstellung der erfindungsgemäßen Verbindungen insbesondere eine gute Möglichkeit zur Bestimmung von Gesamt-Jodthyronin mittels Enzymimmunoassays zur Verfügung gestellt.

Als Markierung in Enzymimmunoassays werden vor allem Peroxidase, alkalische Phosphatase oder β-D-Galactosidase eingesetzt. Insbesondere bei Einsatz von β-D-Galactosidase als Markierungsenzym in Enzymimmunoassays hat sich die Verwendung von erfindungsgemäßen Verbindungen der allgemeinen Formel I zur Verdrängung von Jodthyronin von dieses bindenden Proteinen als vorteilhaft bewährt.

Erfindungsgemäß ganz besonders bevorzugt sind Enzymimmunoassays nach dem IEMA-Prinzip, sogenannte "immuno-enzymometric assays". Hierbei wird der zu untersuchenden Probe enzymmarkierter Antikörper im Überschuß zugegeben. Freies Jodthyronin in der zu untersuchenden Probe bindet an diesen Antikörper. Überschüssiger freier markierter Antikörper wird dann in einem weiteren Schritt abgetrennt, indem festphasenfixiertes Jodthyronin im Überschuß zugegeben wird und so der freie Antikörper an die Festphase fixiert wird. Die Festphase wird dann von der flüssigen Phase getrennt. Anhand der dann entweder in der flüssigen Phase oder auf der Festphase vorliegenden Enzymaktivität, die mittels eines entsprechenden Enzymsubstrates bestimmt werden kann, wird dann die Menge des zu bestimmenden Jodthyronins in der untersuchten Probe ermittelt.

Ein Reagenz zur Durchführung des erfindungsgemäßen Verfahrens zur Bestimmung von Jodthyronin in Gegenwart jodthyroninbindenen Proteins in einer flüssigen Probe ist ebenfalls Gegenstand der vorliegenden Erfindung. Ein solches Reagenz muß eine Substanz enthalten, die zur Verdrängung von Jodthyronin von dieses bindendem Protein geeignet ist und außerdem ein Mittel, das mit freiem, ungebundenem Jodthyronin ein Signal erzeugt, das ein Maß für die Menge an Jodthyronin in der Probe darstellt. Erfindungsgemäß ist ein solches Reagenz dadurch gekennzeichnet, daß es als verdrängende Substanz eine Verbindung der allgemeinen Formel I enthält. Als Mittel zur Bestimmung freien ungebundenen Jodthyronins kann grundsätzlich jedes verwendet werden, das hierzu geeignet ist. Für das erfindungsgemäße Reagenz werden vorzugsweise solche Substanzen als Bestandteile des Mittels zur Bestimmung freien ungebundenen Jodthyronins eingesetzt, die für einen Immunoassay, vorzugsweise einen Enzymimmunoassay notwendig sind. Zur Durchführung eines IEMA-Tests, wie er erfindungsgemäß besonders bevorzugt ist, muß das Reagenz enzymmarkierten Antikörper, trägerfixiertes Jodthyronin und ein der Enzymmarkierung entsprechendes Substrat enthalten, dessen enzymatische Umsetzung zu einem Signal, beispielsweise eine Farbbildung oder Farbveränderung führt, das ein Maß für die Menge an Jodthyronin in der Probe darstellt.

Vorzugsweise enthält das erfindungsgemäße Reagenz eine Puffersubstanz, die in der zu untersuchenden Probe einen pH einstellt, bei dem sowohl die Verdrängungsreaktion mittels der erfindungsgemäßen Substanz der allgemeinen Formel I als auch die Bestimmung von freiem ungebundenem Jodthyronin durchgeführt werden kann. Besonders vorteilhaft wird hierzu eine Puffersubstanz verwendet, die einen pH-Wert zwischen 6 und 8 einstellt.

Das erfindungsgemäße Reagenz kann die Substanz zur Verdrängung von Jodthyronin von dieses bindendem Protein zusammen mit dem Mittel, das mit freiem, ungebundenem Jodthyronin ein Signal erzeugt, das ein Maß für die Menge an Jodthyronin in der untersuchten Probe darstellt, vermischt enthalten. Vorzugsweise werden jedoch die verdrängende Substanz und das Mittel zur Durchführung der Bestimmungsreaktion räumlich getrennt voneinander aufbewahrt.

Das erfindungsgemäße Reagenz kann in Lösung vorliegen. So kann beispielsweise den vorstehenden Ausführungen folgend die Substanz der allgemeinen Formel I zusammen mit dem Mittel zur Bestimmung freien, ungebundenen Jodthyronins in einem gemeinsamen Medium vorliegen. Das erfindungsgemäße Reagenz kann jedoch auch aus mehreren getrennten Lösungen bestehen, die jeweils einzelne Reagenzbestandteile enthalten. Vorzugsweise wird die verdrängende Substanz getrennt von dem Mittel zur Bestimmung freien ungebundenen Jodthyronins vorliegen. Als Lösungsmittel werden bevorzugt solche verwendet, die sowohl zur Lösung der erfindungsgemäßen Verbindungen der allgemeinen Formel I geeignet sind, als auch zur Aufnahme der Komponenten des Mittels zur Bestimmung freien ungebundenen Jodthyronins. In Frage kommen vor allem wässrige Lösungsmittel, besonders bevorzugt Pufferlösungen.

Ein erfindungsgemäßes Reagenz kann auch die Substanz zur Verdrängung von Jodthyronin von dieses bindendem Protein in oder auf einem festen inerten Träger enthalten, während das Mittel zur Bestimmung freien ungebundenen Jodthyronins als Lösung vorliegt. Die umgekehrte Situation ist ebenfalls möglich.

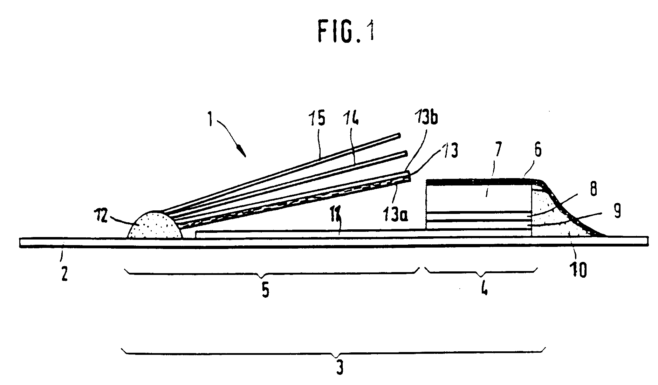

In einer ganz besonders bevorzugten Ausführungsform des erfindungsgemäßen Reagenzes liegt dieses insgesamt trägergebunden vor. Beispielsweise eignet sich hierzu ein Testträger, wie er in Figur 1 dargestellt ist. Ein solcher Testträger ist in EP-A-0 318 777 im Detail beschrieben. Der in Figur 1 dargestellte Testträger 1 hat die prinzipielle Form eines Teststreifens. Es handelt sich um ein hochwertiges Analysesystem, insbesondere für die Durchführung immunologischer Bestimmungen. Auf einer Basisschicht 2 befindet sich der insgesamt mit 3 bezeichnete Testbereich, der sich nur über einen Teil der Länge der Basisschicht 2 erstreckt. Der Testbereich 3 läßt sich in eine Probenaufgabezone 4 und in eine Auswertezone 5 unterteilen. In der Probenaufgabezone 4 erkennt man von oben nach unten ein Abdecknetz 6, eine Erythrozytenabtrennschicht 7 und zwei Reagenzschichten 8 und 9, die mit einem schmelzkleberstreifen 10 an der Basisschicht 2 befestigt sind.

Eine Flüssigkeitstransportschicht 11 aus einem saugfähigen Material, die ebenfalls mit dem schmelzkleberstreifen 10 fixiert ist, erstreckt sich aus der Probenaufgabezone 4 in die Auswertezone 5. Über dem nicht von den Schichten 6 bis 9 bedeckten Bereich der Flüssigkeitstransportschicht 11 befinden sich drei Schichten, die mit einem schmelzkleberstreifen 12 an der Basisschicht 2 derartig befestigt sind, daß sie ohne äußeren Druck schräg von dieser abstehen und sie nicht berühren. Es handelt sich um eine Testschicht 13 mit immobilisiertem Analyt oder Analytanalogon, um eine dritte Reagenzschicht 14 und um eine Abdeckfolie 15.

Der dargestellte bevorzugte Testträger ist insbesondere zur Durchführung immunologischer Bestimmungen geeignet, die dem sogenannten IEMA-Prinzip nachgebildet sind. Soll in einer Probe enthaltenes Jodthyronin (beispielsweise T4) bestimmt werden, so läuft die Analyse mit dem in Figur 1 dargestellten Testträger folgendermaßen ab:
Ein Tropfen Blut (etwa 30 µl) wird oberhalb der Erythrozytenabtrennschicht auf das Abdecknetz 6 aufgegeben und durchdringt die Erythrozytenabtrennschicht 7, die beispielsweise gemäß dem US-Patent 4,477,575 ausgebildet sein kann. Das so erhaltene Serum dringt durch Schicht 8 in Schicht 9 ein. Schicht 8 enthält eine erfindungsgemäße Verbindung der allgemeinen Formel I, so daß hier die von Blut abgeleitete Probe die Substanz aufnimmt, die Jodthyronin von entsprechenden Bindeproteinen verdrängt und so eine Bestimmung des gesamten Jodthyronins in der Probe erlaubt. Schicht 9 enthält einen enzymatisch markierten Antikörper für Jodthyronin im Überschuß gegenüber der maximalen Jodthyroninkonzentration in der Probe. Dieses Antikörper-Enzymkonjugat (AkE) wird von dem eindringenden Serum gelöst. Dabei bilden sich Komplexe zwischen dem AkE und dem Jodthyronin, die mit J-AkE bezeichnet werden. Da das AkE im Überschuß vorhanden ist, bleibt bei Einstellung des Gleichgewichts freies Konjugat AkE übrig. Aufgabe der Schicht 13 ist es, dieses den weiteren Nachweis störende AkE durch eine immunologische Bindung zu beseitigen. Sie wird deshalb auch als immunologische Trennschicht bezeichnet. Sie enthält Jodthyronin oder ein Jodthyroninanalogon in trägerfixierter Form, wobei die Trägerfixierung mit Hilfe von anorganischen Trägerpartikeln erfolgt, wie sie beispielsweise in EP-A-0 318 777 beschrieben sind. Als Trägerpartikel können zweckmäßigerweise SiO₂-Teilchen verwendet werden, wie sie beispielsweise als Silica-Gele für chromatographische Zwecke angeboten werden. Besonders bevorzugt ist jedoch die Verwendung von Titandioxid.

Nach Ablauf einer vorbestimmten Inkubationszeit, in der die Verdrängung gebundenen Jodthyronins von entsprechenden Bindeproteinen in der Probe erfolgt und sich ein Gleichgewicht zwischen freiem, ungebundenem Jodthyronin und AkE einstellt, wird von oben ein Druck auf die Schichten 13 bis 15 ausgeübt. Dies kann manuell oder - wie beispielsweise in der EP-A-0 129 220 beschrieben - mit Hilfe eines Gerätbauteils mechanisch geschehen. Durch das Andrücken kommt die immunologische Abtrennschicht 13 mit der Flüssigkeitstransportschicht 11 in Kontakt und die darin enthaltenen Bestandteile dringen in die Schicht 13 vor. Dabei koppeln die nicht komplexierten AkE an das fixierte Jodthyronin an, während die J-AkE-Komplexe ungehindert weiter vordringen können.

Die Reagenzschicht 14 enthält ein farbbildendes Substrat für das Markierungsenzym. Wenn die Flüssigkeit das Substrat erreicht hat, katalysiert das Enzym der freien J-AkE-Komplexe die Farbreaktion des Substrats. Die Änderungsgeschwindigkeit des Farbumschlages ist deswegen ein Maß für die bei der Reagenzschicht 14 angelangten freien Komplexe J-AkE. Diese wiederum sind ein Maß für das in der Probe enthaltene Jodthyronin.

Mögliche Materialien für die einzelnen Schichten des Testträgers gemäß Figur 1 können EP-A-0 318 777 entnommen werden.

Im folgenden wird die Erfindung anhand von Beispielen näher erläutert.

### Beispiel 1

### 2-Hydroxy-4-methoxydiphenylmethan-5-sulfonsäure Natrium-Salz

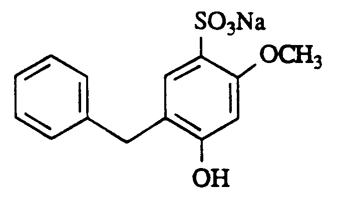

a) 2-Hydroxy-4-methoxybenzophenon-5-sulfonsäure (Uvinul MS-40 der Firma BASF, Ludwigshafen/Rhein, Deutschland; 74 g, 240 mMol) in Wasser (1200 ml) wird mit Natriumcyanoborhydrid (60 g, 950 mMol) in Wasser (700 ml) versetzt und bei Raumtemperatur gerührt. Durch kontinuierliche Zugabe von 2 N Salzsäure wird der pH-Wert der Lösung während 4 Stunden bei 3,5 gehalten und anschließend durch weiteren Zusatz von Salzsäure auf pH 2 gebracht. Danach wird durch die auf 50 °C erwärmte Lösung im Verlauf von 6 Stunden Luft hindurch geleitet zum vollständigen Entfernen der bei der Reaktion freigesetzten Blausäure. Die Lösung wird im Vakuum eingeengt und der Rückstand mit Methanol diggeriert. Der ungelöste Anteil (Kochsalz) wird abgesaugt und das Filtrat zum Ausfällen des Produkts in Ether eingetragen. Durch abermaliges Lösen in warmem Ethanol und Ausfällen in Diethylether wird die Titelverbindung rein erhalten: Ausbeute 70 g (92 %); Fp. > 270 °C; R_{F} 0,3 [Kieselgel; Fließmittel: Isopropanol/n-Butylacetat/Wasser/25 % wässriger Ammoniak = 10:6:3:1 (v/v/v/v)].
b) Alternativ kann die Titelverbindung auch folgendermaßen hergestellt werden:
   2-Hydroxy-4-methoxybenzophenon-5-sulfonsäure (Uvinul MS-40 der Firma BASF, Ludwigshafen/Rhein, Deutschland; 5 g, 16 mMol) in Methanol (500 ml) wird in Gegenwart von Palladium/Kohle (10 %, 1 g) während 6 Stunden bei Raumtemperatur unter Wasserstoff-Atmosphäre gerührt. Nach Absaugen des Katalysators wird mit 1 N methanolischer Natriummethylat-Lösung versetzt, die Lösung im Vakuum eingeengt (Restvolumen ca. 100 ml) und zum Ausfällen des Produkts mit Diethylether (1000 ml) versetzt; Ausbeute 4 g (79 %); Fp. > 270 °C; R_{F} 0,3 [Kieselgel; Fließmittel: Isopropanol/n-Butylacetat/Wasser/25 % wässriger Ammoniak = 10:6:3:1 (v/v/v/v)].

### Beispiel 2

### 2,2'-Dihydroxy-4,4'-dimethoxydiphenylmethan-5,5'-disulfonsäure Di-Natrium-Salz

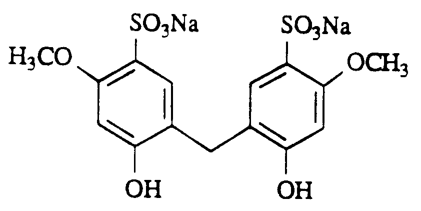

In analoger Weise wie in Beispiel 1 wird 2,2'-Dihydroxy-4,4'-dimethoxybenzophenon-5,5'-disulfonsäure Di-Natrium-Salz (Uvinul DS-49 der Firma BASF, Ludwigshafen/Rhein, Deutschland; 144 g, 0,3 mMol) mit Natriumcyanoborhydrid (57 g, 900 mMol) in Wasser (3000 ml) bei Raumtemperatur während 8 Stunden deoxygeniert, wobei der pH-Wert durch ständige Zugabe von 2 N Salzsäure (Verbrauch insgesamt ca. 250 ml) bei 3,5 gehalten wird. Aufarbeitung wie zuvor ergibt die gewünschte Verbindung; Ausbeute 103 g (74 %); Fp. > 260 °C; R_{F} 0,75 [HPTLC-RP 18, Firma Merck, Darmstadt, Deutschland; Fließmittel: Ethanol/Wasser = 7:3 (v/v)].

### Beispiel 3

### 2-Hydroxy-4-methoxydiphenylcarbinol-5-sulfonsäure Ammonium-Salz

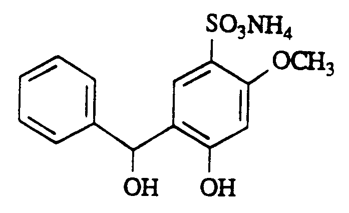

2-Hydroxy-4-methoxybenzophenon-5-sulfonsäure (Uvinul MS-40 der Firma BASF, Ludwigshafen/Rhein, Deutschland; 15,4 g, 50 mMol) in Methanol (800 ml) wird mit Pyridin (4 g, 50 mMol) und PtO₂ (3 g) versetzt und während 24 Stunden bei Raumtemperatur unter Wasserstoff-Atmosphäre gerührt. Nach Abfiltrieren des Katalysators und Einengen des Filtrats wird der Rückstand säulenchromatographisch an Kieselgel aufgereinigt [Fließmittel: Methylenchlorid/Methylethylketon/Methanol/konzentrierter wässriger Ammoniak = 5:1:2:0,3 (v/v/v/v)]. Einengen der Fraktion mit R_{F} 0,12 ergibt die Titelverbindung; Ausbeute 9,5 g (58 %); Fp. 211 °C.

### Beispiel 4

### Verfahren zur Bestimmung von Gesamt-Thyroxin (T4)

A) Testdurchführung
Es werden nacheinander in ein Zentrifugenröhrchen pipettiert:
80 µl Serum mit 0 - 20 µg/dl T4
20 µl Verdrängungsreagenzlösung in Puffer (0 - 30 mM)
Dann wird 2 Minuten gemischt. Anschließend werden zu dieser Mischung
25 µl (Anti-T4-Antikörper)-β-Galactosidase-Konjugat
in Puffer (40 U/ml) gegeben. Der Puffer ist der gleiche wie für das Verdrängungsreagenz. Es wird erneut 2 Minuten gemischt. Dann werden
20 µl Titandioxid-T4-Suspension in Puffer (50 %ig)
zugegeben. Die Titandioxid-T4-Suspension wird wie in EP-A-0 318 777 (Beispiel 1.2) beschrieben, hergestellt. Nach weiterem zweiminütigen Mischen werden feste Bestandteile abzentrifugiert und
20 µl vom Überstand zu
750 µl Chlorphenolrotgalactosid in Puffer (1 mM)
gegeben. Chlorphenolrotgalactosid wurde gemäß EP-A-0 146 866 hergestellt.
Nach kurzem Mischen erfolgt Messung der Extinktionsänderung/min bei 578 nm.
Der benutzte Puffer ist jeweils eine wässrige Lösung (pH = 7,0) folgender Substanzen:

| | |
|---|---|
| KH₂PO₄ | 10 mM |
| MgCl₂ | 5 mM |
| NaCl | 25 mM |

B) Ergebnisse
a) Tabelle 1 zeigt die Abhängigkeit des Meßsignals (Extinktionsänderung pro Minute) von der eingesetzten Verdrängungsreagenz (VR)-Konzentration für die Substanzen 2-Hydroxy-4-methoxydiphenylmethan-5-sulfonsäure (Natriumsalz) (α) und 2,2'-Dihydroxy-4,4'-dimethoxydiphenylmethan-5,5'-disulfonsäure (Dinatriumsalz) (β). Im Vergleich dazu ist der Meßwert eines TBG-freien Serums (Messung ohne VR) mit identischer T4-Konzentration (hier: 5,0 µg/dl im Ansatz) angegeben.

**Tabelle 1**

| 5.0 µg/dl T4 im Gesamtansatz | | | |
|---|---|---|---|
| VR-Konzentration im Ansatz (mM) | Extinktionsänderung/Minute bei Substanz | | TBG-freie Kontrolle |
| | α | β | |
| 0 | 0,13 | 0,20 | 0,63 |
| 2 | 0,35 | 0,45 | |
| 4 | 0,50 | 0,60 | |
| 6 | 0,57 | 0,65 | |
| 8 | 0,60 | 0,66 | |
| 10 | 0,65 | 0,66 | |

Für die Substanz β ist die Verdrängungswirkung ab einer Konzentration von ca. 6 mM vollständig.
Substanz α führt bei einer Konzentration zwischen 8 und 10 mM im Ansatz zum "richtigen" Meßsignal.
b) Tabelle 2 zeigt die Durchführung des Experimentes unter Verwendung von variierenden T4-Konzentrationen im Ansatz mit Substanz β.

**Tabelle 2**

| Substanz β bei verschiedenen T4-Konzentrationen | | | | |
|---|---|---|---|---|
| VR-Konzentration im Ansatz (mM) | Extinktionsänderung/Minute bei T4-Konzentration im Ansatz (µg/dl) | | | |
| | 0,6 | 5,0 | 9,0 | 15,0 |
| 0 | 0,15 | 0,18 | 0,21 | 0,35 |
| 2 | 0,20 | 0,40 | 0,65 | 0,92 |
| 4 | 0,24 | 0,54 | 0,83 | 1,10 |
| 6 | 0,25 | 0,61 | 0,89 | 1,22 |
| 8 | 0,27 | 0,66 | 0,92 | 1,31 |
| 10 | 0,31 | 0,66 | 0,90 | 1,33 |
| 15 | 0,32 | 0,68 | 0,94 | 1,29 |
| 20 | 0,31 | 0,70 | 0,93 | 1,31 |
| 30 | 0,30 | 0,69 | 0,94 | 1,29 |

Danach läßt sich ein Gesamt-T4-Test mit guter Abstufung innerhalb der VR-Konzentrationen von 8 - 30 mM im Ansatz realisieren.

### Beispiel 5

### Testträger zur Bestimmung von Gesamt-Thyroxin (T4)

1. Aufbau und Funktion des Testträgers
Aufbau und Funktion des Testträgers entsprechen Figur 1. Um den Nachweis des gesamten im Serum vorliegenden Thyroxins zu ermöglichen, ist eine Freisetzung des proteingebundenen T4 durch ein geeignetes Verdrängungsreagenz erforderlich, welches sich in der ersten Reagenzschicht 8 befindet.
2. Präparation der Abtrennschicht 13
2.1 Amino-Silanisierung von TiO₂ (TiO₂-Si)
50 g TiO₂ (RN43, Kronos-Titan, Leverkusen, Bundesrepublik Deutschland (BRD)) werden in 1000 ml bidestilliertem Wasser suspendiert und mit 10 ml 3-Aminopropyl-triaethoxysilan (Sigma-Chemie, Deisenhofen, BRD) für 2 Stunden bei 75 °C unter Kontrolle des pH-Wertes (pH 3 - 4) gerührt. Danach wird über eine Glasfritte (G5) abgesaugt und mit bidestilliertem Wasser bis zur pH-Neutralität gewaschen.
2.2 Aufbau der Brückenmoleküle (TiO₂-Si-GA-Cc)
50 g silanisiertes Titandioxid (TiO₂-Si) werden in 250 ml Glutardialdehyd-Lösung (25 %ig in Wasser, Sigma-Chemie, Deisenhofen, BRD) suspendiert und bei pH 7,4 für 12 Stunden gerührt. Danach wird über eine Glasfritte (G5) abgesaugt, zunächst mit bidestilliertem Wasser und dann mit 0,5 M Phosphat-Puffer (pH 7,6) gewaschen. Die feste Phase wird in 225 ml Phosphat-Puffer aufgenommen und 12 Stunden mit 25 ml Crotein-C-Lösung (10 %ig in Phosphat-Puffer, Crotein C von Croda, Nettetal, BRD) gerührt. Zur Absättigung noch freier Aldehyd-Gruppen werden 50 g in Phosphat-Puffer gewaschene Festphase in 500 ml 0,5 M Phosphat-Puffer mit 0,5 M Glycin für 1 Stunde gerührt. Nach intensivem Waschen (bidest. Wasser) wird das Sediment (ca. 50 g) in 450 ml Borat-Puffer (pH 8,5) aufgenommen und mit 50 ml Natriumcyanoborhydrid-Lösung (5 %ig in Borat-Puffer) für 15 Minuten zur Reduktion der Schiff'schen Basen gerührt. Anschließend wird über eine Glasfritte (G5) abgesaugt und mit Phosphat-Puffer und bidestilliertem Wasser gewaschen.
2.3 Ankopplung von BOC-T4-Hydroxysuccinimid (TiO₂-Si-GA-Cc-T4) als Analyt-Analogon
10 g TiO₂ -Si-GA-Cc werden in 200 ml 0,06 M Na₂HPO₄ (pH 8,8) suspendiert und mit 170 ml BOC-T4-Hydroxysuccinimid-Lösung 0,05 %ig in Dioxan) für 2 Stunden im Dunkeln gerührt. Danach wird mit 0,06 M Na₂HPO₄/Dioxan (10:8,5) und mehrfach mit bidestilliertem Wasser gewaschen und abgesaugt.
2.4 Filmbeschichtung auf Gewebe
Eine Beschichtungsmasse mit der Zusammensetzung:

| | |
|---|---|
| Propiofan 70 D (BASF, Ludwigshafen, BRD) | 4 g |
| Brij 35 (Serva, Heidelberg, BRD) 15 %ige Lösung in Gal-Puffer | 1 g |
| Polyox WSR 301 (Union Carbide, New York, USA) 2,5 %ig in Gal-Puffer | 5 g |
| TiO₂-T4-Matrixkopplung, 40 %ige Suspension in Gal-Puffer | 30 g |
| Kieselgur MW 25 | 9 g |
| | 49 g |

Wird auf ein Gewebe PE 812 K⁶ (Schweizerische Seidengazefabrik, Thal, Schweiz) mit einer Naßfilmdicke von 350 µ aufgetragen und getrocknet.
3. Präparation des Verdrängungsreagenz auf der ersten Reagenzschicht 8
200 mM Verdrängungsreagenz (2,2'-Dihydroxy-4,4'-dimethoxy-diphenylmethan-5,5'-disulfonsäure (Dinatriumsalz)) werden in Gal-Puffer gelöst und auf Teebeutelpapier (Schöller und Hösch, Gernsheim, BRD) getränkt.

| | | |
|---|---|---|
| Gal-Puffer: | KH₂PO₄ | 10 mM |
| | MgCL₂ | 5 mM |
| | NaCl | 25 mM |
| | pH | 7,0 |

4. Präparation der zweiten Reagenzschicht 9 als Konjugatschicht
Tränkung der folgenden Lösung auf Gewebe PE 14 100 normal (Schweizer Seidengazefabrik, Thal, Schweiz)

| Zusammensetzung der Tränklösung | |
|---|---|
| Hepes | 50 mM |
| MgCl₂ | 5 mM |
| Trehalose | 1 % |
| Crotein C | 1 % |
| 〈T4〉-βGal-Konjugat | 80 U/ml |
| pH | 6,6 |

5. Präparation der dritten Reagenzschicht 14 als Substratschicht
Tränkung auf Gewebe PE HD-1 (Schweizer Seidengazefabrik, Thal, Schweiz)
Zusammensetzung der Tränklösung: Chlorphenolrotgalactosid (CPRG) (hergestellt gemäß EP-A-0 146 866) 20 mM in Gal-Puffer
6. Ergebnisse
30 µl Serum mit verschiedenen Gesamt-T4-Konzentrationen werden auf Teststreifen aufgetragen und in dem Gerät "Reflotron" der Anmelderin bei 567 nm vermessen:

| µg/dl T4 | % Remission nach 1 Minute |
|---|---|
| 0,55 | 52,25 |
| 6,65 | 44,51 |
| 10,00 | 38,52 |
| 20,90 | 31,48 |

Die erreichbare Abstufung im klinisch relevanten Bereich von 2,0 - 17,0 µg/dl erlaubt eine sehr gute Meßgenauigkeit.

### Beispiel 6

### Beeinflussung der Enyzmaktivität von β-Galactosidase als Markierungsenzym durch Verdrängungsreagenzien

A) Testdurchführung
Es werden nacheinander in ein Zentrifugenröhrchen pipettiert:
80 µl Serum mit 5 µg/dl T4
20 µl Verdrängungsreagenzlösung in Puffer (0 - 40 mM)
Dann wird 2 Minuten gemischt. Anschließend werden zu dieser Mischung
25 µl (Anti-T4-Antikörper)-β-Galactosidase-Konjugat
in Puffer (40 U/ml) gegeben. Der Puffer ist der gleiche wie für das Verdrängungsreagenz. Es wird erneut 2 Minuten gemischt. Dann werden
20 µl dieser Mischung zu
750 µl Chlorphenolrotgalactosid in Puffer (1 mM)
gegeben. Chlorphenolrotgalactosid wurde gemäß EP-A-0 146 866 hergestellt.
Nach kurzem Mischen erfolgt Messung der Extinktionsänderung/min bei 578 nm.
Der benutzte Puffer ist jeweils eine wässrige Lösung (pH = 7,0) folgender Substanzen:

| | |
|---|---|
| KH₂PO₄ | 10 mM |
| MgCl₂ | 5 mM |
| NaCl | 25 mM |

B) Ergebnisse
a) Tabelle 3 zeigt die Abhängigkeit des Meßsignals (Extinktionsänderung pro Minute) als Maß für die Enzymaktivität von der eingesetzten Verdrängungsreagenz (VR)-Konzentration für die Substanzen 2-Hydroxy-4-methoxydiphenylmethan-5-sulfonsäure (Natriumsalz) (α) (vergleiche Beispiel 1), 2,2'-Dihydroxy-4,4'-dimethoxydiphenylmethan-5,5'-disulfonsäure (Dinatriumsalz) (β) (vergleiche Beispiel 2) und 2-Hydroxy-4-methoxybenzophenon-5-sulfonsäure (γ) (Stand der Technik, vergleiche EP-A-0 133 464)

**Tabelle 3**

| VR-Konzentration im Ansatz (mM) | Extinktionsänderung/Minute bei Substanz | | |
|---|---|---|---|
| | α | β | γ |
| 0 | 1,32 | 1,47 | 1,37 |
| 2 | 1,51 | 1,39 | 1,59 |
| 5 | 1,42 | 1,32 | 1,58 |
| 10 | 1,54 | 1,45 | 1,38 |
| 20 | 1,37 | 1,32 | 1,18 |
| 40 | 1,45 | 1,34 | 1,05 |

Während die erfindungsgemäßen Substanzen (α,β) keine Beeinflussung der β-Galactosidase-Aktivität zeigen, wird die Enzymaktivität durch das Verdrängungsreagenz des Standes der Technik (γ) merklich beeinflußt. Bei höheren VR-Konzentrationen nimmt die Enzymaktivität stark ab.

## Patentansprüche

1. Verbindung der allgemeinen Formel I 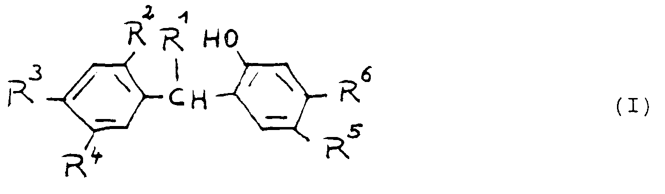 wobei
R¹ Wasserstoff oder Hydroxy,
R² Wasserstoff oder Hydroxy,
R³ Wasserstoff oder Alkoxy,
R⁴ Wasserstoff, CO₂H, SO₃H, PO₃H₂ oder ein Salz der entsprechenden Säure,
R⁵ CO₂H, SO₃H, PO₃H₂ oder ein Salz der entsprechenden Säure und
R⁶ Alkoxy
bedeuten.

2. Verbindung der allgemeinen Formel I gemäß Anspruch 1, dadurch gekennzeichnet, daß
R¹ Wasserstoff oder Hydroxy,
R², R³ und R⁴ Wasserstoff,
R⁵ SO₃H oder ein entsprechendes Salz und
R⁶ Alkoxy
bedeuten.

3. Verbindung der allgemeinen Formel I gemäß Anspruch 1, dadurch gekennzeichnet, daß
R¹ Wasserstoff oder Hydroxy,
R² Hydroxy,
R³ Alkoxy,
R⁴ SO₃H oder ein entsprechendes Salz,
R⁵ SO₃H oder ein entsprechendes Salz und
R⁶ Alkoxy
bedeuten.

4. Verfahren zur Herstellung einer Verbindung der allgemeinen Formel I 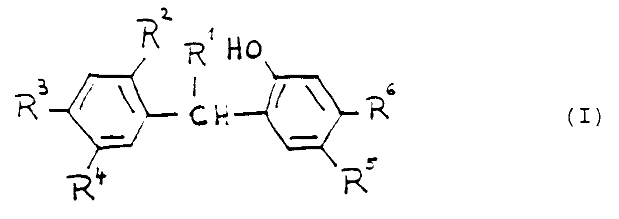 wobei
R¹ Wasserstoff oder Hydroxy,
R² Wasserstoff oder Hydroxy,
R³ Wasserstoff oder Alkoxy,
R⁴ Wasserstoff, CO₂H, SO₃H, PO₃H₂ oder ein Salz der entsprechenden Säure,
R⁵ CO₂H, SO₃H, PO₃H₂ oder ein Salz der entsprechenden Säure und
R⁶ Alkoxy
bedeuten, dadurch gekennzeichnet, daß Verbindungen der allgemeinen Formel II 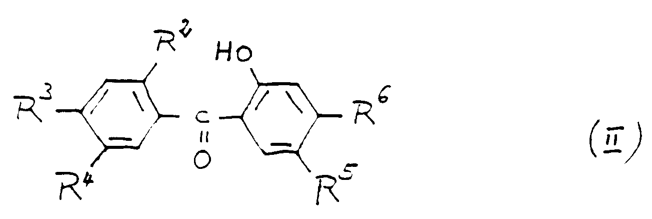 in der
R² - R⁶ die Bedeutung wie für Formel I angegeben haben,
reduziert werden.

5. Verwendung einer Verbindung der allgemeinen Formel I 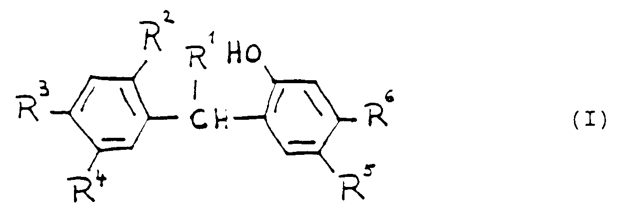 wobei
R¹ Wasserstoff oder Hydroxy,
R² Wasserstoff oder Hydroxy,
R³ Wasserstoff oder Alkoxy,
R⁴ Wasserstoff, CO₂H, SO₃H, PO₃H₂ oder ein Salz der entsprechenden Säure,
R⁵ CO₂H, SO₃H, PO₃H₂ oder ein Salz der entsprechenden Säure und
R⁶ Alkoxy
bedeuten zur Verdrängung von Jodthyroninen von diesen bindenden Proteinen.

6. Verfahren zur Bestimmung eines Jodthyronins in Gegenwart Jodthyronin bindenden Proteins in einer flüssigen Probe durch Zugabe einer Substanz, die an Protein gebundenes Jodthyronin von dort verdrängt und freisetzt und
Bestimmung des gesamten Jodthyronins in der Probe, insbesondere mittels einer immunologischen Methode, dadurch gekennzeichnet, daß als verdrängende Substanz eine Verbindung der allgemeinen Formel I 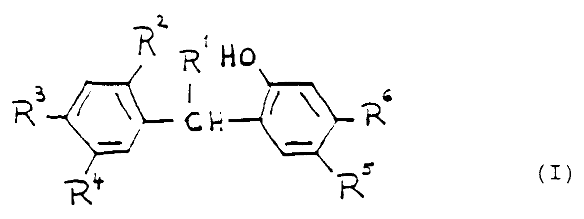 wobei
R¹ Wasserstoff oder Hydroxy,
R² Wasserstoff oder Hydroxy,
R³ Wasserstoff oder Alkoxy,
R⁴ Wasserstoff, CO₂H, SO₃H, PO₃H₂ oder ein Salz der entsprechenden Säure,
R⁵ CO₂H, SO₃H, PO₃H₂ oder ein Salz der entsprechenden Säure und
R⁶ Alkoxy
bedeuten, zu der Probe zugesetzt wird.

7. Reagenz zur Bestimmung von Jodthyronin in einer Probe,
enthaltend eine Substanz zur Verdrängung von Jodthyronin von dieses bindendem Protein und ein Mittel, das mit freiem, ungebundenem Jodthyronin ein Signal erzeugt, das ein Maß für die Menge an Jodthyronin in der Probe darstellt, dadurch gekennzeichnet, daß es als verdrängende Substanz eine Verbindung der allgemeinen Formel I enthält, 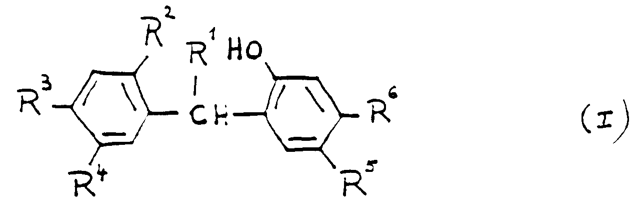 wobei
R¹ Wasserstoff oder Hydroxy,
R² Wasserstoff oder Hydroxy,
R³ Wasserstoff oder Alkoxy,
R⁴ Wasserstoff, CO₂H, SO₃H, PO₃H₂ oder ein Salz der entsprechenden Säure,
R⁵ CO₂H, SO₃H, PO₃H₂ oder ein Salz der entsprechenden Säure und
R⁶ Alkoxy
bedeuten.

8. Reagenz gemäß Anspruch 7, dadurch gekennzeichnet, daß es die Substanz zur Verdrängung von Jodthyronin von dieses bindendem Protein räumlich getrennt von dem Mittel, das mit freiem, ungebundenem Jodthyronin ein Signal erzeugt, das ein Maß für die Menge an Jodthyronin in der untersuchten Probe darstellt, enthält.

9. Reagenz gemäß Anspruch 7 oder 8, dadurch gekennzeichnet, daß die Substanz zur Verdrängung von Jodthyronin von dieses bindendem Protein in oder auf einem festen Träger enthalten ist.

10. Reagenz gemäß einem der Ansprüche 7 bis 9, dadurch gekennzeichnet, daß es trägergebunden vorliegt.

## Claims

1. Compounds of the general formula I 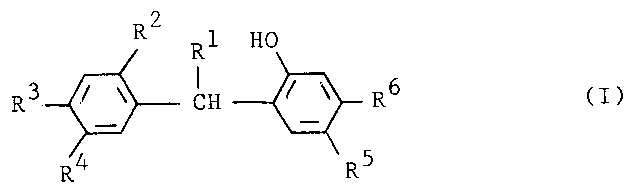 whereby R¹ signifies hydrogen or hydroxyl, R² hydrogen or hydroxyl, R³ hydrogen or alkoxy, R⁴ hydrogen, CO₂H, SO₃H, PO₃H₂ or a salt of the corresponding acid, R⁵ CO₂H, SO₃H, PO₃H₂ or a salt of the corresponding acid and R⁶ alkoxy.

2. Compounds of the general formula I according to claim 1, characterised in that R¹ signifies hydrogen or hydroxyl, R², R³ and R⁴ hydrogen, R⁵ SO₃H or a corresponding salt and R⁶ alkoxy.

3. Compounds of the general formula I according to claim 1, characterised in that R¹ signifies hydrogen or hydroxyl, R² hydroxyl, R³ alkoxy, R⁴ SO₃H or a corresponding salt, R⁵ SO₃H or a corresponding salt and R⁶ alkoxy.

4. Process for the preparation of a compound of the general formula I 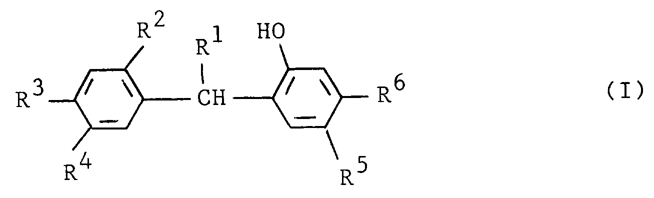 whereby R¹ signifies hydrogen or hydroxyl, R² hydrogen or hydroxyl, R³ hydrogen or alkoxy, R⁴ hydrogen, CO₂H, SO₃H, PO₃H₂ or a salt of the corresponding acid R⁵ CO₂H, SO₃H, PO₃H₂ or a salt of the corresponding acid and R⁶ alkoxy, characterised in that compounds of the general formula II 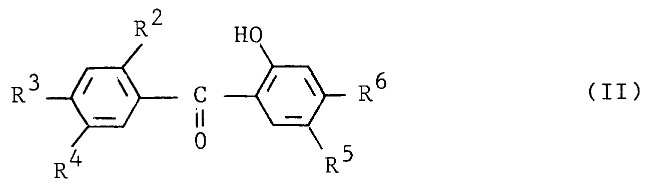 in which R² - R⁶ have the meaning as given for formula I, are reduced.

5. Use of a compound of the general formula I 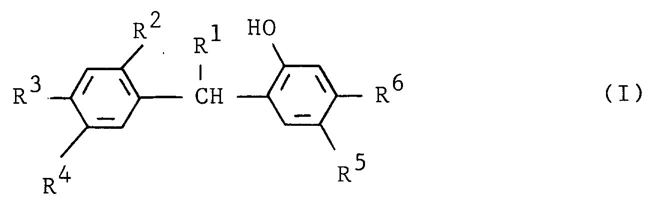 whereby R¹ signifies hydrogen or hydroxyl, R² hydrogen or hydroxyl, R³ hydrogen or alkoxy, R⁴ hydrogen, CO₂H, SO₃H, PO₃H₂ or a salt of the corresponding acid, R⁵ CO₂H, SO₃H, PO₃H₂ or a salt of the corresponding acid and R⁶ alkoxy, for the displacement of iodothyronines from proteins binding these.

6. Process for the determination of an iodothyronine in the presence of iodothyronine-binding protein in a liquid sample by addition of a substance which displaces and liberates iodothyronine bound to protein from there and determination of the total iodothyronine in the sample, especially by means of an immunological method, characterised in that, as displacing substance, to the sample is added a compound of the general formula I 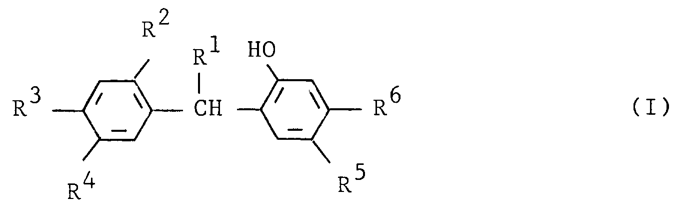 whereby R¹ signifies hydrogen or hydroxyl, R² hydrogen or hydroxyl, R³ hydrogen or alkoxy, R⁴ hydrogen, CO₂H, SO₃H, PO₃H₂ or a salt of the corresponding acid, R⁵ CO₂H, SO₃H, PO₃H₂ or a salt of the corresponding acid and R⁶ alkoxy.

7. Reagent for the determination of iodothyronine in a sample, containing a substance for the displacement of iodothyronine from protein binding this and an agent which, with free non-bound iodothyronine, produces a signal which represents a measure for the amount of iodothyronine in the sample, characterised in that, as displacing substance, it contains a compound of the general formula I 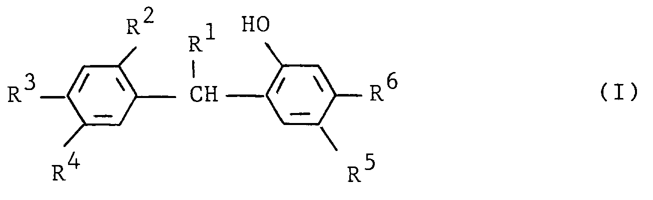 whereby R¹ signifies hydrogen or hydroxyl, R² hydrogen or hydroxyl, R³ hydrogen or alkoxy, R⁴ hydrogen, CO₂H, SO₃H, PO₃H₂ or a salt of the corresponding acid, R⁵ CO₂H, SO₃H, PO₃H₂ or a salt of the corresponding acid and R⁶ alkoxy.

8. Reagent according to claim 7, characterised in that it contains the substance for the displacement of iodothyronine from protein binding this separate from the agent which, with free non-bound iodothyronine, produces a signal which represents a measure for the amount of iodothyronine in the investigated sample.

9. Reagent according to claim 7 or 8, characterised in that the substance for the displacement of iodothyronine from protein binding this is contained in or on a solid carrier.

10. Reagent according to one of claims 7 to 9, characterised in that it is present carrier-bound.

## Revendications

1. Composé de formule générale I 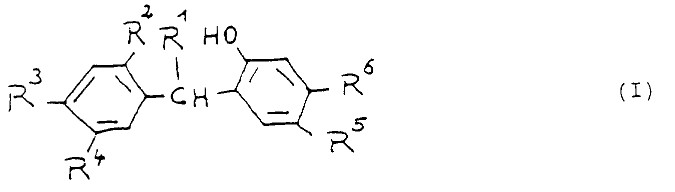 dans laquelle
R¹ représente un atome d'hydrogène ou un groupe hydroxy,
R² représente un atome d'hydrogène ou un groupe hydroxy,
R³ représente un atome d'hydrogène ou un groupe alcoxy,
R⁴ représente un atome d'hydrogène, un groupe CO₂H, SO₃H, PO₃H₂ ou un sel de l'acide correspondant,
R⁵ représente un groupe CO₂H, SO₃H, PO₃H₂ ou un sel de l'acide correspondant, et
R⁶ représente un groupe alcoxy.

2. Composé de formule générale I selon la revendication 1, caractérisé en ce que
R¹ représente un atome d'hydrogène ou un groupe hydroxy,
R² ,R³ et R⁴ représentent un atome d'hydrogène,
R⁵ représente un groupe SO₃H ou un sel correspondant, et
R⁶ représente un groupe alcoxy.

3. Composé de formule générale I selon la revendication 1, caractérisé en ce que
R¹ représente un atome d'hydrogène ou un groupe hydroxy,
R² représente un groupe hydroxy,
R³ représente un groupe alcoxy,
R⁴ représente un groupe SO₃H ou un sel correspondant,
R⁵ représente un groupe SO₃H ou un sel correspondant, et
R⁶ représente un groupe alcoxy.

4. Procédé pour la préparation d'un composé de formule générale I 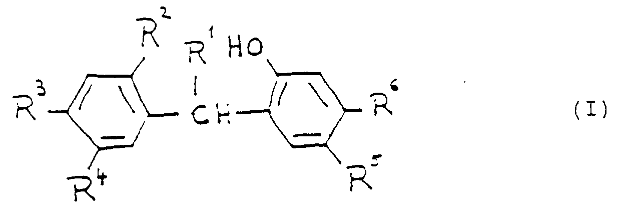 dans laquelle
R¹ représente un atome d'hydrogène ou un groupe hydroxy,
R² représente un atome d'hydrogène ou un groupe hydroxy,
R³ représente un atome d'hydrogène ou un groupe alcoxy,
R⁴ représente un atome d'hydrogène, un groupe CO₂H, SO₃H, PO₃H₂ ou un sel de l'acide correspondant,
R⁵ représente un groupe CO₂H, SO₃H, PO₃H₂ ou un sel de l'acide correspondant, et
R⁶ représente un groupe alcoxy,
caractérisé en ce que l'on réduit des composés de formule générale II 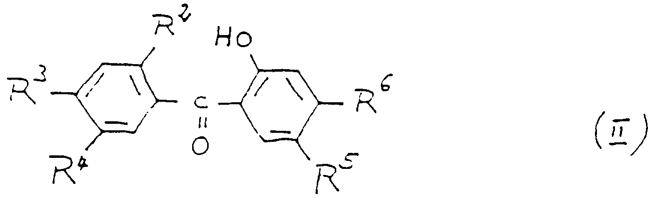 dans laquelle
R² - R⁶ ont les significations indiquées à propos de la formule I.

5. Utilisation d'un composé de formule générale I 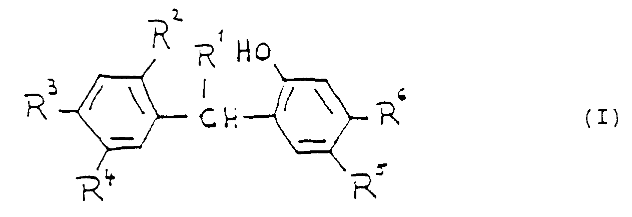 dans laquelle
R¹ représente un atome d'hydrogène ou un groupe hydroxy,
R² représente un atome d'hydrogène ou un groupe hydroxy,
R³ représente un atome d'hydrogène ou un groupe alcoxy,
R⁴ représente un atome d'hydrogène, un groupe CO₂H, SO₃H, PO₃H₂ ou un sel de l'acide correspondant,
R⁵ représente un groupe CO₂H, SO₃H, PO₃H₂ ou un sel de l'acide correspondant, et
R⁶ représente un groupe alcoxy,
pour le déplacement d'iodothyronines à partir des protéines auxquelles elles sont liées.

6. Procédé pour la détermination d'une iodothyronine en présence de protéines fixant les iodothyronines, dans un échantillon liquide, par addition d'une substance qui déplace la iodothyronine à partir de la protéine à laquelle elle est liée et la libère, et
pour la détermination de la teneur totale en iodothyronines de l'échantillon, en particulier au moyen d'un procédé immunologique, caractérisé en ce que comme substance de déplacement, on ajoute à l'échantillon un composé de formule générale I 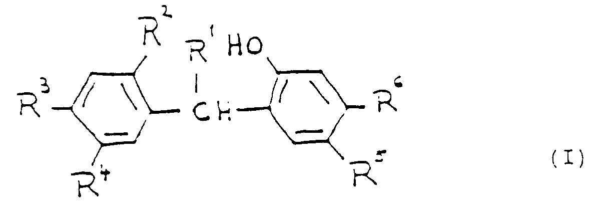 dans laquelle
R¹ représente un atome d'hydrogène ou un groupe hydroxy,
R² représente un atome d'hydrogène ou un groupe hydroxy,
R³ représente un atome d'hydrogène ou un groupe alcoxy,
R⁴ représente un atome d'hydrogène, un groupe CO₂H, SO₃H, PO₃H₂ ou un sel de l'acide correspondant,
R⁵ représente un groupe CO₂H, SO₃H, PO₃H₂ ou un sel de l'acide correspondant, et
R⁶ représente un groupe alcoxy.

7. Réactif pour la détermination des iodothyronines dans un échantillon, contenant une substance destinée à déplacer la iodothyronine à partir de protéines auxquelles elle est liée, et un agent qui, avec la iodothyronine libre, non liée, produit un signal qui constitue une mesure de la quantité d'iodothyronine dans l'échantillon, caractérisé en ce que comme substance de déplacement, il contient un composé de formule générale I 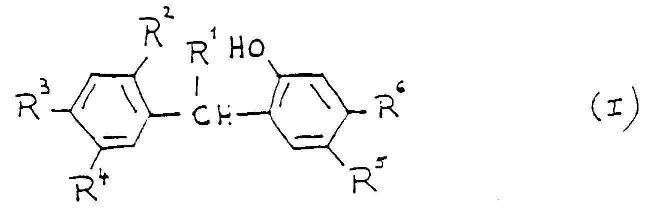 dans laquelle
R¹ représente un atome d'hydrogène ou un groupe hydroxy,
R² représente un atome d'hydrogène ou un groupe hydroxy,
R³ représente un atome d'hydrogène ou un groupe alcoxy,
R⁴ représente un atome d'hydrogène, un groupe CO₂H, SO₃H, PO₃H₂ ou un sel de l'acide correspondant,
R⁵ représente un groupe CO₂H, SO₃H, PO₃H₂ ou un sel de l'acide correspondant, et
R⁶ représente un groupe alcoxy.

8. Réactif selon la revendication 7, caractérisé en ce qu'il contient la substance destinée à déplacer la iodothyronine à partir de protéines auxquelles elle est liée, séparément de l'agent qui, avec la iodothyronine libre, non liée, produit un signal qui constitue une mesure de la quantité d'iodothyronine dans l'échantillon analysé.

9. Réactif selon la revendication 7 ou 8, caractérisé en ce que la substance, destinée à déplacer la iodothyronine à partir de protéines auxquelles elle est liée, est contenue dans, ou fixée sur, un support solide.

10. Réactif selon les revendications 7 à 9, caractérisé en ce qu'il est lié à un support.
